# FASCICULE DE BREVET EUROPEEN

(11) **EP 4 034 232 B1**
(45) Date de publication et mention de la délivrance du brevet: **01.11.2023**
(21) Numéro de dépôt: 20776183.4
(22) Date de dépôt: 28.09.2020
(51) Int. Cl.: A61N 5/06

(54) **SYSTÈME D'ACUPUNCTURE LASER COMPORTANT UN REPOSE-TÊTE ET UN SUPPORT POUR DES BOÎTIERS ÉMETTEURS LASER**
LASER-AKUPUNKTURSYSTEM, DAS EINE KOPFSTÜTZE UND EIN STÜTZELEMENT FÜR LASER-SENDERGEHÄUSE UMFASST
LASER ACUPUNCTURE SYSTEM COMPRISING A HEADREST AND A SUPPORT MEMBER FOR LASER TRANSMITTER HOUSINGS

(30) Priorité: 26.09.2019 FR 1910623
(43) Date de publication de la demande: 03.08.2022
(73) Titulaire: BE.TA +, 35830 Betton (FR)
(72) Inventeur: MOIGNE, Pascal, 35620 TEILLAY (FR); RENAULT, Joël, 35830 BETTON (FR)
(74) Mandataire: Cabinet Le Guen Maillet
(86) Numéro de dépôt international: PCT/EP2020/077118
(87) Numéro de publication internationale: WO 2021/058823

(56) Documents cités:
- EP-A1- 1 163 887
- EP-B1- 1 163 887
- DE-C1- 19 710 797
- DE-U1- 20 309 976
- US-A1- 2014 316 492

## Description

### DOMAINE TECHNIQUE

La présente invention concerne un système d'acupuncture laser comportant un repose-tête et un support pour des boîtiers émetteurs laser.

### ETAT DE LA TECHNIQUE ANTERIEURE

Il est connu d'utiliser l'acupuncture pour traiter certains problèmes. L'acupuncture peut être basée sur l'utilisation d'épingles qui sont plantées dans la peau du sujet, ou de faisceaux laser qui sont appliqués contre la peau du sujet.

Le faisceau laser est généralement embarqué dans un boîtier qui est tenu à la main par un praticien qui le maintient en face du point de la peau devant être soumis à ce faisceau laser. Bien qu'un tel boîtier donne de bons résultats, il est nécessaire de trouver une autre méthode pour pouvoir appliquer plusieurs faisceaux laser simultanément au même sujet.

Un système d'acupuncture laser est connu par exemple de DE19710797C1.

### EXPOSE DE L'INVENTION

Un objet de la présente invention est de proposer un système d'acupuncture laser comportant un repose-tête et un support pour des boîtiers émetteurs laser, où le système permet d'éclairer simultanément plusieurs points de la peau du sujet sans que le praticien ait besoin de maintenir les boîtiers émetteurs laser en position.

A cet effet, est proposé un système d'acupuncture laser comportant une base destinée à reposer sur un plan, un repose-tête maintenu à la base, un arceau monté mobile en rotation sur la base au-dessus du repose-tête autour de charnières auto-stables, et au moins un boîtier émetteur laser comportant un socle maintenu à l'arceau, une tige qui présente une première extrémité solidaire du socle et une deuxième extrémité, et un émetteur laser qui est fixé à la deuxième extrémité de la tige, où la tige déformable et auto-stable.

Un tel système permet de s'affranchir de la tenue des boîtiers émetteurs laser par le praticien. Avantageusement, le système d'acupuncture laser comporte deux charnières, où une charnière est fixée entre chaque extrémité de l'arceau et la base.

Avantageusement, le repose-tête est réglable en position par rapport à la base.

Avantageusement, le socle est fixé de manière amovible à l'arceau.

Avantageusement, le socle présente une rainure dans laquelle s'introduit l'arceau et le socle comporte des moyens de fixation qui assurent le maintien en position du socle sur l'arceau tout en autorisant le démontage du socle de l'arceau.

Avantageusement, la rainure présente une forme de biseau qui se resserre vers le fond de la rainure.

Avantageusement, le système d'acupuncture laser comporte un coussin d'appui dorsal maintenu à la base.

Avantageusement, le coussin d'appui dorsal est réglable en position par rapport à la base.

Avantageusement, chaque émetteur laser est équipé d'une diode qui émet dans le visible et dont le faisceau lumineux est coaxial avec le faisceau laser émis par ledit émetteur laser.

Avantageusement, le repose-tête prend la forme d'un U dont les branches sont destinées à venir sur les côtés de la tête du sujet et dont la base est destinée à être positionnée sous le cou du sujet.

### BREVE DESCRIPTION DES DESSINS

Les caractéristiques de l'invention mentionnées ci-dessus, ainsi que d'autres, apparaîtront plus clairement à la lecture de la description suivante d'au moins un exemple de réalisation, ladite description étant faite en relation avec les dessins joints, parmi lesquels :
[Fig. 1] est une vue en perspective d'un système d'acupuncture laser selon l'invention,
[Fig. 2] est une vue en perspective du système d'acupuncture laser de la Fig. 1 dans une autre position, et
[Fig. 3] est une vue en perspective d'un boîtier émetteur laser.

### EXPOSE DETAILLE DE MODES DE REALISATION

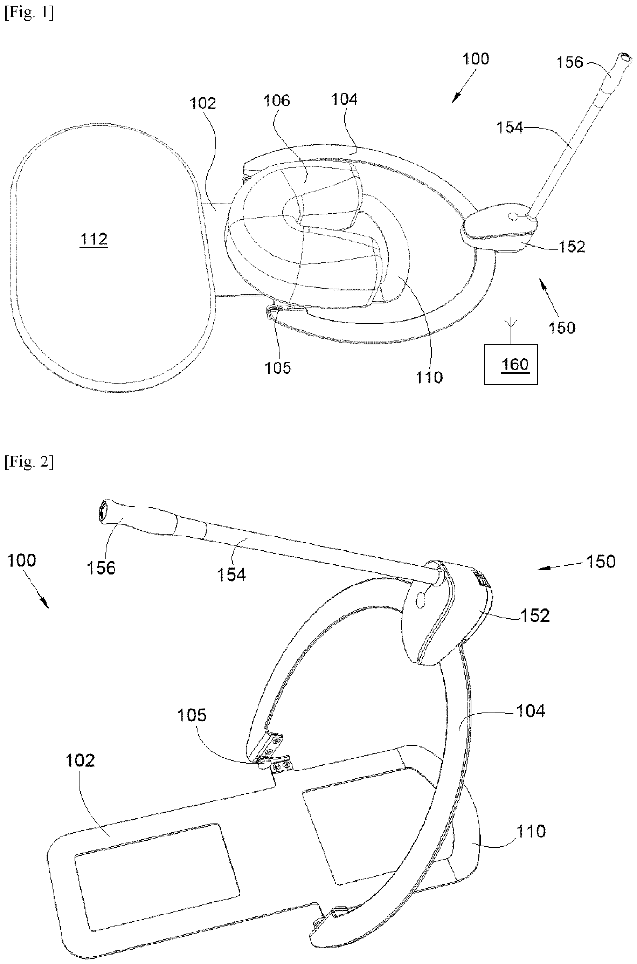

La Fig. 1 et la Fig. 2 montrent un système d'acupuncture laser 100 comportant une base 102 et un repose-tête 106.

Le système d'acupuncture laser 100 comporte également un arceau 104 qui est monté mobile en rotation sur la base 102 au-dessus du repose-tête 106 autour de deux charnières 105.

Chaque charnière 105 est auto-stable, c'est-à-dire qu'elle ne bouge pas tant qu'une personne n'agit pas sur l'arceau 104. Il peut s'agir par exemple de charnières 105 qui sont suffisamment serrées.

La base 102 est destinée à reposer sur un plan et le sujet à traiter est allongé sur la base 102 et pose sa tête sur le repose-tête 106.

L'arceau 104 peut être librement positionné au-dessus du repose-tête 106 et donc de la tête du sujet, c'est-à-dire qu'il peut prendre différentes positions au-dessus de la tête du sujet. Dans le mode de réalisation de l'invention présenté ici, l'arceau 104 est mobile jusqu'à une position allongée (Fig. 1) qui permet de rendre le système d'acupuncture laser 100 plat pour faciliter le rangement et la mise en place de la tête du sujet sur le repose-tête 106.

Sur la Fig. 2, le repose-tête 106 n'est pas représenté pour laisser voir plus clairement les charnières 105. Une charnière 105 est fixée entre chaque extrémité de l'arceau 104 et la base 102.

Le repose-tête 106 est ainsi maintenu à la base 102 entre les deux extrémités de l'arceau 104.

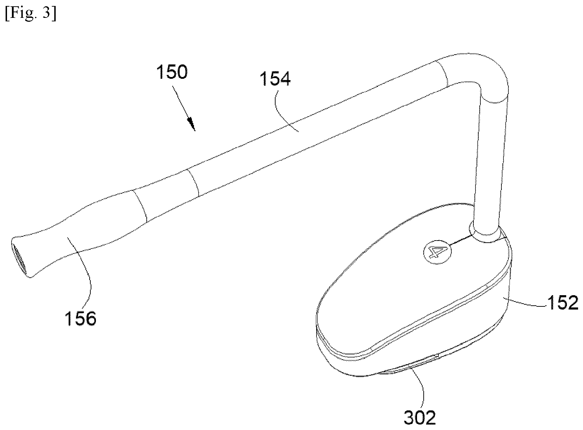

Le système d'acupuncture laser 100 comporte également au moins un boîtier émetteur laser 150 qui est également montré en Fig. 3.

Le boîtier émetteur laser 150 comporte un socle 152 qui est maintenu à l'arceau 104, une tige 154 qui présente une première extrémité solidaire du socle 152 et une deuxième extrémité, et un émetteur laser 156 qui est fixé à la deuxième extrémité de la tige 154. L'émetteur laser 156 est par exemple une diode laser qui émet un faisceau laser.

La tige 154 est souple et déformable et également auto-stable, c'est-à-dire que lorsqu'une forme lui est donnée, elle reste en position tant qu'une autre position ne lui est pas imposée, comme cela est représenté en Fig. 3.

La tige 154 est par exemple une tige articulée métallique, par exemple, en aluminium. Ainsi lorsqu'un sujet place sa tête sur le repose-tête 106, l'arceau 104 est positionné avec les boîtiers émetteurs laser 150, et chaque tige 154 est réglée pour pointer le faisceau laser émis par son émetteur laser 156 à l'endroit approprié sur la tête. Il n'est alors plus nécessaire de tenir les boîtiers émetteurs laser 150.

Chaque socle 152 contient les éléments nécessaires au fonctionnement de l'émetteur laser 156 comme par exemple une source d'énergie électrique comme une batterie, un moyen d'activation comme un interrupteur.

Il est également possible de commander à distance les émetteurs laser 156 des différents boîtiers émetteur lasers 150. A cette fin, le système d'acupuncture laser 100 comporte une unité de contrôle 160 qui peut être programmée par un praticien pour allumer/éteindre séquentiellement certains émetteurs laser 156. L'unité de contrôle 160 peut être un ordinateur équipé d'un logiciel approprié. L'unité de contrôle 160 peut également envoyer la puissance et le temps de traitement de chaque émetteur laser 156. L'unité de contrôle 160 peut également recevoir des informations de chaque émetteur laser 156, par exemple des informations montrant le bon fonctionnement dudit émetteur laser 156.

Chaque émetteur laser 156 peut être équipé d'une diode qui émet dans le visible et dont le faisceau lumineux est coaxial avec le faisceau laser émis par ledit émetteur laser 156 afin de faciliter le positionnement dudit émetteur laser 156.

A cette fin, la liaison entre l'unité de contrôle 160 et chaque boîtier émetteur laser 150 peut être filaire, mais elle est préférentiellement sans fil par exemple à travers la technologie Bluetooth^{®}, Wifi^{®} ou autre. Le socle 152 et l'unité de contrôle 160 contiennent alors chacun un émetteur/récepteur radio pour communiquer l'un avec l'autre.

Le repose-tête 106 est avantageusement réglable en position par rapport à la base 102 pour faciliter l'adaptation de sa position par rapport à la base 102 en fonction du sujet à traiter. Selon un mode de réalisation particulier, la base 102 est métallique et le repose-tête 106 est équipé d'aimants qui coopèrent avec la base 102 pour assurer une fixation réglable du repose-tête 106 et il est alors possible de changer le repose-tête 106 en fonction du sujet à traiter.

Pour faciliter l'accès à l'arrière de la tête du sujet, le repose-tête 106 prend la forme d'un U dont les branches viennent sur les côtés de la tête du sujet pour la maintenir et dont la base est positionnée sous le cou du sujet pour le maintenir, en laissant ainsi libre la partie arrière de la tête.

Dans le mode de réalisation de l'invention, le socle 152 est fixé de manière amovible à l'arceau 104. Ici, le socle 152 présente une rainure 302 dans laquelle s'introduit l'arceau 104. Le socle 152 comporte des moyens de fixation qui assurent le maintien en position du socle 152 sur l'arceau 104 tout en autorisant le démontage du socle 152 de l'arceau 104. La rainure 302 assure une meilleure stabilité du socle 152.

Les moyens de fixation prennent par exemple la forme de vis de serrage ou d'aimants qui coopèrent avec le matériau métallique de l'arceau 104.

En outre, avantageusement, la rainure 302 présente une forme de biseau qui se resserre vers le fond de la rainure 302 afin de pincer l'arceau 104.

Dans le mode de réalisation de l'invention présenté sur les Figs. 1 et 2, la base 102 comporte une extension 110 qui s'étend au-delà du repose-tête 106, c'est-à-dire au-delà de la tête du sujet par rapport à son corps. L'extension 110 est configurée pour recevoir des boîtiers émetteurs laser 150.

Pour assurer un positionnement agréable du sujet, le système d'acupuncture laser 100 comporte également un coussin d'appui dorsal 112 qui est maintenu à la base 102 et avantageusement il est réglable en position par rapport à la base 102. Comme pour le repose-tête 106, il peut être équipé d'aimants qui coopèrent avec la base 102 pour assurer une fixation réglable.

Dans le mode de réalisation de l'invention présenté sur les Figs. 1 et 2, l'arceau 104 est circulaire, mais il peut prendre une autre forme par exemple rectangulaire ou ovale.

## Revendications

1. Système d'acupuncture laser (100) comportant :
- une base (102) destinée à reposer sur un plan,
- un repose-tête (106) maintenu à la base (102),
- des charnières (105) auto-stables,
- un arceau (104) monté mobile en rotation sur la base (102) au-dessus du repose-tête (106) autour des charnières (105) auto-stables, et
- au moins un boîtier émetteur laser (150) comportant un socle (152) maintenu à l'arceau (104), une tige (154) qui présente une première extrémité solidaire du socle (152) et une deuxième extrémité, et un émetteur laser (156) qui est fixé à la deuxième extrémité de la tige (154), où la tige (154) est déformable et auto-stable.

2. Système d'acupuncture laser (100) selon la revendication 1, **caractérisé en ce qu'**il comporte deux charnières (105), où une charnière (105) est fixée entre chaque extrémité de l'arceau (104) et la base (102).

3. Système d'acupuncture laser (100) selon l'une des revendications 1 ou 2, **caractérisé en ce que** le repose-tête (106) est réglable en position par rapport à la base (102).

4. Système d'acupuncture laser (100) selon l'une des revendications 1 à 3, **caractérisé en ce que** le socle (152) est fixé de manière amovible à l'arceau (104).

5. Système d'acupuncture laser (100) selon la revendication 4, **caractérisé en ce que** le socle (152) présente une rainure (302) dans laquelle s'introduit l'arceau (104) et le socle (152) comporte des moyens de fixation qui assurent le maintien en position du socle (152) sur l'arceau (104) tout en autorisant le démontage du socle (152) de l'arceau (104).

6. Système d'acupuncture laser (100) selon la revendication 5, **caractérisé en ce que** la rainure (302) présente une forme de biseau qui se resserre vers le fond de la rainure (302).

7. Système d'acupuncture laser (100) selon l'une des revendications 1 à 6, **caractérisé en ce qu'**il comporte un coussin d'appui dorsal (112) maintenu à la base (102).

8. Système d'acupuncture laser (100) selon la revendication 7, **caractérisé en ce que** le coussin d'appui dorsal (112) est réglable en position par rapport à la base (102).

9. Système d'acupuncture laser (100) selon l'une des revendications 1 à 8, **caractérisé en ce que** chaque émetteur laser (156) est équipé d'une diode qui émet dans le visible et dont le faisceau lumineux est coaxial avec le faisceau laser émis par ledit émetteur laser (156).

10. Système d'acupuncture laser (100) selon l'une des revendications 1 à 9, **caractérisé en ce que** le repose-tête (106) prend la forme d'un U dont les branches sont destinées à venir sur les côtés de la tête du sujet et dont la base est destinée à être positionnée sous le cou du sujet.

## Patentansprüche

1. Laserakupunktur-System (100) mit:
- einer Basis (102), die dazu bestimmt ist, auf einer Ebene zu ruhen,
- einer Kopfstütze (106), die an der Basis (102) gehalten wird,
- selbststabilisierenden Scharnieren (105),
- einem Bügel (104), der an der Basis (102) oberhalb der Kopfstütze (106) um die selbststabilisierenden Scharniere (105) angebracht ist, und drehbar ist, und
- mindestens einem Lasersendergehäuse (150) mit einem Sockel (152), der am Bügel (104) gehalten wird, einer Stange (154), die ein erstes Ende, welches fest mit dem Sockel (152) verbunden ist, und ein zweites Ende aufweist, und einem Lasersender (156), der am zweiten Ende der Stange (154) befestigt ist, wobei die Stange (154) verformbar und selbststabilisierend ist.

2. Laserakupunktur-System (100) nach Anspruch 1, **dadurch gekennzeichnet, dass** es zwei Scharniere (105) aufweist, wobei ein Scharnier (105) zwischen jedem Ende des Bügels (104) und der Basis (102) befestigt ist.

3. Laserakupunktur-System (100) nach einem der Ansprüche 1 oder 2, **dadurch gekennzeichnet, dass** die Kopfstütze (106) in ihrer Position relativ zu der Basis (102) einstellbar ist.

4. Laserakupunktur-System (100) nach einem der Ansprüche 1 bis 3, **dadurch gekennzeichnet, dass** der Sockel (152) so am Bügel (104) befestigt ist, dass sie abnehmbar ist.

5. Laserakupunktur-System (100) nach Anspruch 4, **dadurch gekennzeichnet, dass** der Sockel (152) eine Nut (302) aufweist, in die der Bügel (104) eingeführt wird, und der Sockel (152) Befestigungsvorrichtungen umfasst, die sicherstellen, dass der Sockel (152) auf dem Bügel (104) in Position gehalten wird, während sie gleichzeitig die Demontage des Sockels (152) vom Bügel (104) ermöglichen.

6. Laserakupunktur-System (100) nach Anspruch 5, **dadurch gekennzeichnet, dass** die Nut (302) eine abgeschrägte Form aufweist, die sich zur Unterseite der Nut (302) hin verengt.

7. Laserakupunktur-System (100) nach einem der Ansprüche 1 bis 6, **dadurch gekennzeichnet, dass** es ein Rückenstützkissen (112) aufweist, das an der Basis (102) gehalten wird.

8. Laserakupunktur-System (100) nach Anspruch 7, **dadurch gekennzeichnet, dass** das Rückenstützkissen (112) in seiner Position in Bezug auf die Basis (102) einstellbar ist.

9. Laserakupunktur-System (100) nach einem der Ansprüche 1 bis 8, **dadurch gekennzeichnet, dass** jeder Lasersender (156) mit einer Diode ausgestattet ist, die im sichtbaren Bereich emittiert und deren Lichtstrahl koaxial zu dem von dem Lasersender (156) emittierten Laserstrahl ist.

10. Laserakupunktur-System (100) nach einem der Ansprüche 1 bis 9, **dadurch gekennzeichnet, dass** die Kopfstütze (106) die Form eines U hat, dessen Bügel dazu bestimmt sind, an die Seiten des Kopfes des Kunden zu kommen, und dessen Basis dazu bestimmt ist, unter dem Hals des Kunden positioniert zu werden.

## Claims

1. Laser acupuncture system (100) comprising:
- a base (102) intended to rest on a plane,
- a headrest (106) held at the base (102),
- self-stabilizing hinges (105),
- an arch (104) rotatably mounted on the base (102) above the headrest (106) around the self-stabilizing hinges (105), and
- at least one laser emitter box (150) comprising a stand (152) held to the arch (104), a rod (154) which has a first end secured to the stand (152) and a second end, and a laser emitter (156) which is attached to the second end of the rod (154), where the rod (154) is deformable and self-stabilising.

2. Laser acupuncture system (100) according to claim 1, **characterized in that** it comprises two hinges (105), where a hinge (105) is fixed between each end of the arch (104) and the base (102).

3. Laser acupuncture system (100) according to one of claims 1 or 2, characterized 15 in that the headrest (106) is adjustable in position relative to the base (102).

4. Laser acupuncture system (100) according to one of claims 1 to 3, **characterized in that** the stand (152) is removably fixed to the arch (104).

5. Laser acupuncture system (100) according to claim 4, **characterized in that** the stand (152) has a groove (302) into which the arch (104) is inserted and the stand (152) comprises fastening means which hold the stand (152) in position on the arch (104) while allowing the dismantling of the stand (152) of the arch (104).

6. Laser acupuncture system (100) according to claim 5, **characterized in that** the groove (302) has a bevel shape that narrows towards the bottom of the groove (302).

7. Laser acupuncture system (100) according to one of claims 1 to 6, **characterized in that** it comprises a back support cushion (112) maintained at the base (102).

8. Laser acupuncture system (100) according to claim 7, **characterized in that** the back support cushion (112) is adjustable in position relative to the base (102).

9. Laser acupuncture system (100) according to one of Claims 1 to 8, **characterized in that** each laser transmitter (156) is equipped with a diode that emits in the visible and of which the light beam is coaxial with the laser beam emitted by said laser transmitter (156).

10. Laser acupuncture system (100) according to one of claims 1 to 9, **characterized in that** the headrest (106) takes the form of a U whose branches are intended to come on the sides of the head of the subject and whose base is intended to be positioned under the subject's neck.
